# EUROPÄISCHE PATENTSCHRIFT

(11) **EP 2 254 735 B1**
(45) Veröffentlichungstag und Bekanntmachung des Hinweises auf die Patenterteilung: **13.07.2011**
(21) Anmeldenummer: 09724141.8
(22) Anmeldetag: 20.03.2009
(51) Int. Cl.: B25J 9/16, A61B 19/00

(54) **OPERATIONS-ASSISTENZ-SYSTEM ZUR FÜHRUNG EINES CHIRURGISCHEN HILFSINSTRUMENTES**
SURGERY ASSISTANCE SYSTEM FOR GUIDING A SURGICAL INSTRUMENT
SYSTÈME D'ASSISTANCE OPÉRATOIRE POUR GUIDER UN INSTRUMENT CHIRURGICAL AUXILIAIRE

(30) Priorität: 28.03.2008 DE 102008016146
(43) Veröffentlichungstag der Anmeldung: 01.12.2010
(73) Patentinhaber: Aktormed GmbH, 93092 Barbing (DE)
(72) Erfinder: GEIGER, Robert, 94516 Metten (DE); SCHERR, Jürgen, 93464 Tiefenbach (DE)
(74) Vertreter: Graf Glück Habersack Kritzenberger
(86) Internationale Anmeldenummer: PCT/DE2009/000387
(87) Internationale Veröffentlichungsnummer: WO 2009/117989

(56) Entgegenhaltungen:
- US-A- 5 911 036
- US-A- 6 024 695
- US-A1- 2005 183 532
- US-A1- 2007 173 977

## Beschreibung

Die Erfindung bezieht sich auf ein Operations-Assistenz-System zur Führung eines chirurgischen Hilfsinstrumentes, insbesondere eines ein Endoskop aufweisendes Kamerasystems abhängig von der manuellen Betätigung zumindest einer Funktionstaste eines Bedienelements gemäß dem Oberbegriff des Patentanspruches 1.

Aus der DE 103 52 197 ist beispielsweise ein Operations-Assistenz-System bekannt, das bei minimal invasiven Eingriffen oder Operationen zur Führung von chirurgischen Hilfsinstrumenten, wie z.B. Kamerasystemen usw. Verwendung findet.

Die DE 10 2004 052 753 A1 betrifft ein Verfahren zur Steuerung der Nachführung zumindest eines chirurgischen Hilfsinstruments in Bezug auf ein führendes chirurgisches Instrument mittels eines Operations-Assistenz-Systems, bei dem bei einem minimal invasiven Eingriff die aktuellen Positionsdaten eines ersten Sensorelementes in einem dreidimensionalen Messraum ermittelt werden, wobei das erste Sensorelement an einem aus dem Patientenkörper herausragenden Abschnitt des führenden chirurgischen Instruments vorgesehen ist. Abhängig von dem ermittelten Positionsdaten des führenden chirurgischen Instruments werden die Positionsdaten des nach zuführenden chirurgischen Hilfsinstrument berechnet und dieses über das Operations-Assistenz-System entsprechend nachgeführt.

Aus der Veröffentlichung "Visual Servoing For Automatic and Uncalibrated Needle Placement For Percutaneous Procedures" von Nassir Navab et. al., IEEE, 2000 ist ein Verfahren zur bildgesteuerten Führung einer Nadel oder eines chirurgischen Instrumentes im Rahmen einer perkutanen Operationsmethode bekannt, bei der die aktuelle Position der zu führenden Nadel bzw. des zu führenden chirurgischen Instrumentes über ein bildgebendes Verfahren dem Operateur angezeigt wird.

Die WO 03/041057 A2 beschreibt ein System und ein zugehöriges Verfahren zur bildgeführten Instrumentensteuerung über eine Robotereinheit bzw. eines Roboterarmes, an dem ein zu führendes Instrument angeordnet ist. Ein System zur Führung eines chirurgischen Instrumentes bei chirurgischen Operationen, insbesondere Wirbelsäulenoperationen ist auch bereits der US 2003/0187351 zu entnehmen.

Nachteilig sind jedoch die aus dem Stand der Technik bekannten Operations-Assistenz-Systeme in Bezug auf den Bewegungsraum des mittels des Operations-Assistenz-Systems motorisch geführten Hilfsinstruments beschränkt.

Aufgabe der Erfindung ist es, ein Operations-Assistenz-System zur Führung eines chirurgischen Hilfsinstruments, insbesondere eines ein Endoskop aufweisendes Kamerasystems anzugeben, welches sich durch eine präzisere Instrumentenführung, einen vergrößerten Bewegungsraum des geführten chirurgischen Hilfsinstruments und eine hohe Bedienerfreundlichkeit auszeichnet. Die Aufgabe wird ausgehend von den Merkmalen des Oberbegriffes des Patentanspruches 1 durch dessen kennzeichnende Merkmale gelöst.

Ein wesentlicher Aspekt des erfindungsgemäßen Operations-Assistenz-Systems ist darin zu sehen, dass eine Steuer- und Auswerteroutine vorgesehen ist, die zur Ermittlung der Neigungswinkel des chirurgischen Hilfsinstrumentes in Bezug auf die durch den Trokarpunkt verlaufende Raumachse des kartesischen Patientenkoordinatensystem und zum Vergleich der ermittelte Neigungswinkel mit einem vorgegebenen Sollneigungswinkel ausgebildet ist, wobei bei einem Überschreiten des Sollneigungswinkels durch den ermittelten Neigungswinkel die Spitze des chirurgischen Hilfsinstrumentes auf einer die Spitze aufnehmenden Teilkugeloberfläche geführt wird und bei einem Unterschreiten des Sollneigungswinkel durch den ermittelten Neigungswinkel die Spitze des chirurgischen Hilfsinstrumentes entlang einer durch die Spitze des Hilfsinstrumentes verlaufenden Tangente an die Teilkugeloberfläche geführt wird.

In einer vorteilhaften Variante des erfindungsgemäßen Operations-Assistenz-Systems wird bei einem Überschreiten des Sollneigungswinkels durch den ermittelten Neigungswinkel die Spitze des Hilfsinstrumentes auf einer den Trokarpunkt konzentrisch umgebenden Kreisbahn geführt wird.

Vorteilhaft wird der Sollneigungswinkels zwischen 10° und 25°, vorzugsweise zwischen 15° und 20° gewählt. Hierdurch wird eine präzise Führung des chirurgischen Hilfsinstruments auch bei äußerst geringen Neigungswinkeln und einer nahezu senkrecht zur x-y-Ebene verlaufenden Längsachse des Hilfsinstruments ermöglicht. Nach Erreichen eines ausreichenden Abstandes der Spitze des Hilfsinstruments von der z- Achse des kartesischen Patientenkoordinatensystems erfolgt eine automatische Umschaltung von der Tangentensteuerung auf die Kugelsteuerung. Abhängig von der gewählten Steuerungsart erfolgt die Berechnung der Führungsbahn zur Führung der Spitze des chirurgischen Hilfsinstruments im Patientenkörper.

Weiterbildungen, Vorteile und Anwendungsmöglichkeiten des erfindungsgemäßen Operations-Assistenz-Systems sind Gegenstand der abhängigen Ansprüche.

Die Erfindung wird im Folgenden anhand der Figuren an einem Ausführungsbeispiel näher erläutert. Es zeigen:
- Fig. 1: in perspektivischer Darstellung ein Operations-Assistenz-Systems;
- Fig. 2: ein schematisches Blockschaltbild eines Computersystems mit angeschlossenen Bedienelement und Monitoreinheit;
- Fig. 3: einen Querschnitt durch einen Patientenkörper, bei dem in den Operationsraum die Spitze eines Endoskops eines Kamerasystems eingeführt ist;
- Fig. 4: eine Draufsicht auf die x-y-Ebene eines kartesischen Patientenkoordinatensystems bei einer Führung der Spitze eines Endoskops entlang einer Tangente;
- Fig. 5: ein Flussdiagramm einer Auswerte- und Steuerroutine zur Ansteuerung des Operations-Assistenz-Systems;
- Fig. 6: ein Flussdiagramm einer Registrierungsroutine und

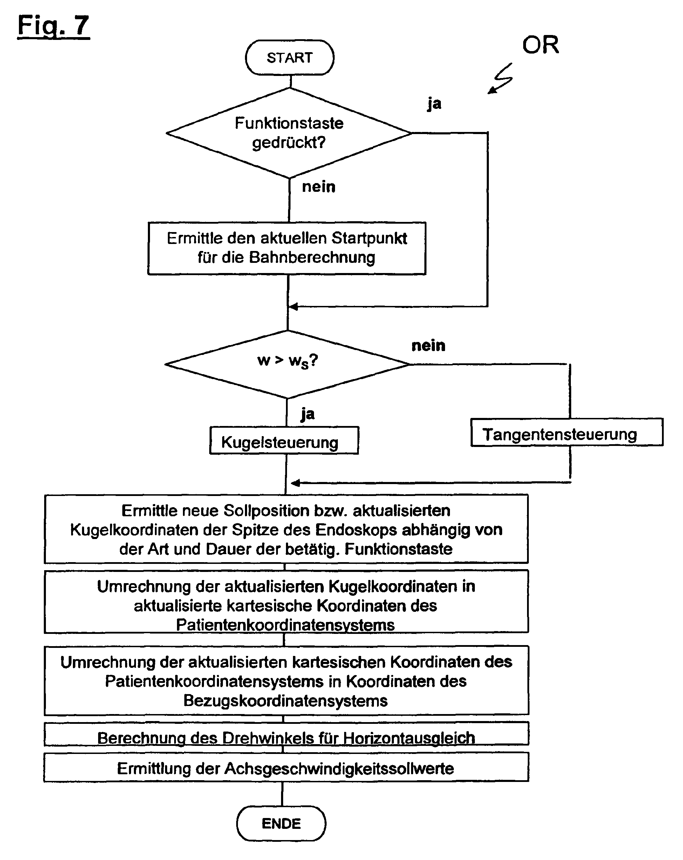

- Fig. 7: ein Flussdiagramm einer Operationsroutine.

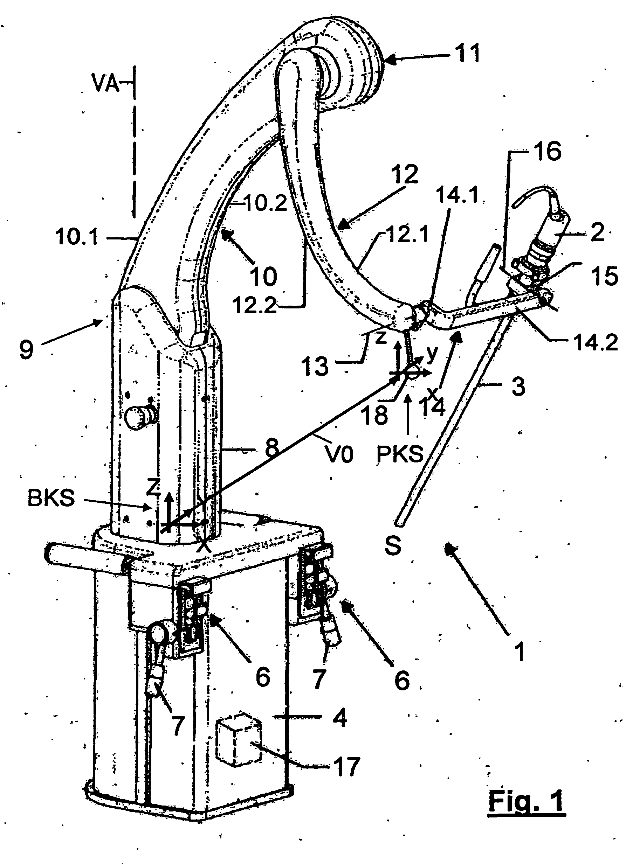

Das in Figur 1 mit 1 bezeichnete Operations-Assistenz-System für medizinische Interventionen, insbesondere minimal invasive Operationen ist zur Führung von chirurgischen Hilfsinstrumenten, beispielsweise zum Führen eines ein Endoskop 3 aufweisendes Kamerasystem 2 ausgebildet. Das chirurgische Hilfsinstrument, insbesondere das Endoskop 3 des Kamerasystems 2 wird hierbei über eine kleinformatige Operationsöffnung ("Trokar") oder einen Trokarpunkt T in einen Operationsraum 19 innerhalb eines Patientenkörpers 20 eingeführt.

Das Operations-Assistenz-System 1 weist ein Gehäuse 4 auf, in welchem die wesentlichen Funktions- und Steuerelemente untergebracht sind und welches beispielsweise seitlich an einem Operationstisch 5 befestigbar ist, und zwar bei der darstellten Ausführungsform über zwei manuell betätigbare Klemmeinrichtungen 6, die an einer gemeinsamen Seite des Gehäuses 4 gegeneinander versetzt vorgesehen sind und zu deren Betätigung jeweils ein Schwenkhebel 7 vorgesehen ist. Zu Ansteuerung des Operations-Assistenz-Systems 1 und zur Verarbeitung der mittels des Kamerasystems 2 erzeugten Bilddaten ist das Operations-Assistenz-Systems 1 an ein Computersystem CS angeschlossen.

Über die Oberseite des Gehäuses 4 des Operations-Assistenz-Systems 1 steht eine Tragsäule 8 vor, welche durch einen im Gehäuse 4 untergebrachten elektromotorischen Antrieb (nicht in den Figuren dargestellt) um eine vertikale Achse VA (Hochachse) gesteuert dreh- bzw. schwenkbar ist. Im Bereich des oberen Endes der Tragsäule 8 ist an dieser über ein Gelenk 9 das eine bzw. untere Ende eines inneren Armes 10 angelenkt.

Das im Wesentlichen aus einem Gelenkbolzen und zugehörigen Lagern bestehende Gelenk 9 ist dabei so ausgebildet, dass der Arm 10 mit seinem unteren Ende in das obere, offene Ende der Tragsäule 8 hineinreicht, und dort von der Tragsäule 8 gabelartig umschlossen ist. Der als Hohlkörper aus einem leichten, aber stabilen Werkstoff, z.B. aus faserverstärktem Kunststoff, beispielsweise aus glasfaserverstärktem oder kohlefaserverstärktem Kunststoff hergestellte Arm 10 ist in Armlängsrichtung gekrümmt ausgebildet, und zwar an der Armoberseite 10.1 konvex und an der Armunterseite 10.2 konkav.

An dem der Tragsäule 8 entfernt liegenden Ende des Armes 10 ist an diesem über ein Gelenk 11 der äußere Arm 12 angelenkt. Das Gelenk 11 besteht im Wesentlichen aus Lagerelementen und einem Gelenkbolzen, der beidendig aus dem Arm 10 herausgeführt ist. Mit einem aus dem Arm 10 herausgeführten Ende des Gelenkbolzens ist das eine Ende des Armes 12 in geeigneter Weise über ein nicht dargestelltes Verbindungssystem drehfest, aber abnehmbar verbunden. Der ebenfalls als Hohlkörper aus einem leichten, aber stabilen Werkstoff, z.B. aus faserverstärktem Kunststoff, beispielsweise aus glasfaserverstärktem oder kohlefaserverstärktem Kunststoff ausgebildete Arm 12 ist in Armlängsrichtung gekrümmt, und zwar an der Armoberseite 12.1 konkav und an der Armunterseite 12.2 konvex.

An dem dem Arm 10 entfernt liegenden Ende des Armes 12 ist mittels eines weiteren Gelenks 13 der kürzere Schenkel 14.1 eines L-förmigen Werkzeugträgers 14 frei drehbar gelagert, und zwar um eine Achse parallel zur Längserstreckung des längeren Schenkels 14.2 dieses Werkzeugträgers 14. An dem dem kürzeren Schenkel 14.1 entfernt liegenden Ende des längeren Schenkels 14.2 ist am Werkzeugträger 14 ein als Klipp ausgebildeter Werkzeughalter 15 vorgesehen, an welchem das Endoskop 3 durch Verrasten lösbar befestigt ist und der seinerseits mittels eines Gelenks 16 um eine Achse senkrecht zur Achse des Gelenks 13 dreh- und schwenkbar ist.

Bei der dargestellten Ausführungsform sind die Schwenkachsen der Gelenke 9, 11 und 13 parallel oder im Wesentlichen parallel zueinander orientierte horizontale oder im Wesentlichen horizontale Achsen. Die Gelenke 13 und 16 sind freie Gelenke, d.h. diese Gelenke ermöglichen ein freies Schwenken des Werkzeugträgers 14 relativ zum Arm 12 bzw. des Halters 15 relativ zum Werkzeugträger 14. Die Gelenke 9 und 11 bilden hingegen jeweils gesteuerte Achsen für eine gesteuerte hydraulisch betätigte Schwenkbewegungen, und zwar unter Verwendung jeweils eines hydraulischen Antriebs, der über eine im Gehäuse 4 untergebrachte Steuereinrichtung 17 gesteuert wird. Die Steuereinrichtung 17 ist hierbei zur Erzeugung von Steuersignalen zur Ansteuerung der unterschiedlichen Antriebe der Kinematik des Operations-Assistenz-Systems 1 in einem kartesischen Koordinatensystem vorgesehen. Das der Kinematik des Operations-Assistenz-Systems 1 zugeordnete kartesische Koordinatensystem mit den Raumachsen X, Y, Z wird nachfolgend als Bezugskoordinatensystem BKS bezeichnet.

Zur Einstellung der Null- oder Ausgangsposition des Operations-Assistenz-Systems 1 bzw. des Endoskops 3 in Bezug auf die Operationsöffnung bzw. den Trokarpunkt T, durch die bzw. den das Endoskop 3 in den Operationsraum 19 eingeführt werden soll, ist am freien Ende des Armes 12 ein stabförmiger Registriertaster 18 vorgesehen, der an seinem vom Arm 12 wegstehenden Ende einen Kugelkopf aufweist. Der Kugelkopf des starr am Arm 12 vorgesehenen Registriertasters 18 weist hierbei eine definierte Lage zu den Bezugs- oder Ausgangsstellungen der gesteuerten Achsen des Operations-Assistenz-Systems 1 bzw. zum Bezugskoordinatensystem BKS auf. Vor der Operation wird das Operations-Assistenz-Systems 1 dadurch registriert, dass der Registriertaster 18 an denjenigen Bereich des auf dem OP-Tisch bereits platzierten Patienten geführt wird, an dem die Operationsöffnung bzw. der Trokarpunkt T zum Einführen des Endoskops 3 vorgesehen ist. Hierdurch wird ein Patientenkoordinatensystem PKS festgelegt, welches einen vorgegebenen Offsetvektor Vo zum Bezugskoordinatensystem BKS aufweist. Die kartesischen Koordinaten X0 Y0, Z0 des Offsetvektors V0 im Bezugskoordinatensystem BKS werden vorzugsweise in der Steuereinrichtung 17 gespeichert.

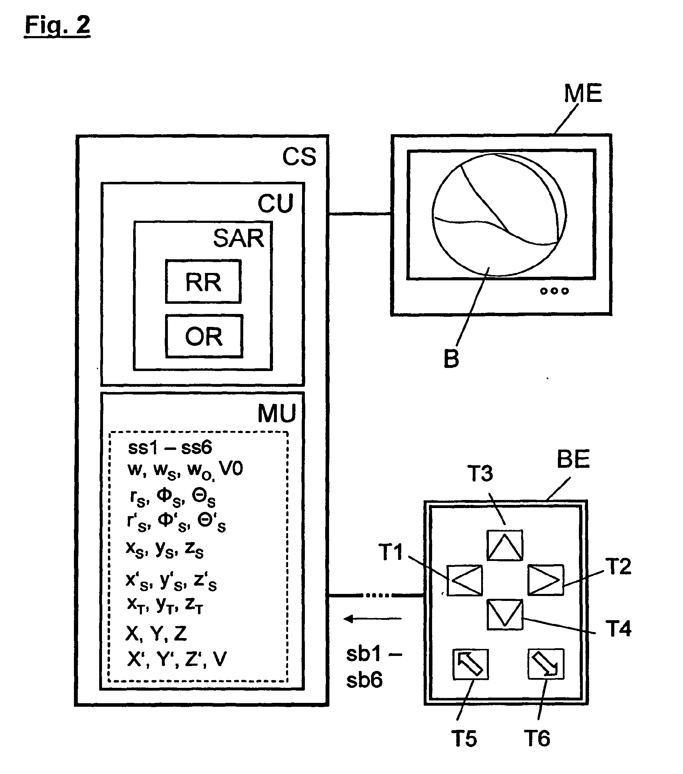

Zur Steuerung der Bewegung des Hilfsinstrumentes, insbesondere des Endoskops 3 des Operations-Assistenz-Systems 1 ist zumindest ein Bedienelement BE vorgesehen, welches beispielsweise als Fußschalter, Joystick oder Handbedienteil ausgebildet sein kann. In Figur 2 ist beispielsweise ein derartiges Bedienelement BE dargestellt, welches beispielsweise sechs Funktionstasten T1 - T6 umfasst. Das Bedienelement BE ist an das Computersystem CS angeschlossen, dass zumindest eine Steuereinheit CU und eine Speichereinheit MU aufweist.

Zur Auswertung und Weiterverarbeitung der mittels des Kamerasystems 2 erzeugten Bilddaten sowie zur Steuerung der Bewegung des Hilfsinstrumentes, insbesondere des Endoskops 3 mittels des Bedienelementes BE ist das Computersystem CS mit der Steuereinrichtung 17 sowie der Kamera 2 des Operations-Assistenz-Systems 1 verbunden, und zwar vorzugsweise über in den Figuren nicht dargestellte Schnittstelleneinheiten oder ein Bussystem, beispielsweise ein CAN-Bussystem.

Zur Darstellung der durch das Kamerasystem 2 erzeugten und an das Computersystem CS zur Weiterverarbeitung übertragenen Bilder B ist an das Computersystem CS eine Monitoreinheit ME angeschlossen. Auf der Monitoreinheit ME wird das über das Endoskop 3 durch das Kamerasystem 2 erfasste Bild B dargestellt, welches dem Bediener des Operations-Assistenz-Systems 1 den vor der Spitze S des Endoskops 3 befindlichen Bereich des Operationsraumes 19 zeigt.

Hierbei wird bei Inbetriebnahme und Registrierung des Operations-Assistenz-Systems 1 der Bildhorizont des auf der Monitoreinheit ME dargestellten Bildes B durch Drehen des Kamerasystems 2 um dessen Längsachse L derart eingestellt, dass dies der natürlichen Sichtweise des Bedieners entspricht. Der dadurch manuell eingestellte Offsetwinkel wo wird durch eine in der Steuereinheit CU ausgeführte Steuer- und Auswerteroutine SAR erfasst und in der Speichereinheit MU gespeichert. Mittels des ermittelten Offsetwinkels wo wird sichergestellt, dass im Rahmen der Führung des Endoskops 3 im Operationsraum 19 der Bildhorizont des auf der Monitoreinheit ME dargestellten Bildes B nahezu konstant bleibt.

Die Steuer- und Auswerteroutine SAR ist hierbei derart ausgebildet, dass die von dem Bedienelement BE durch Betätigen der Funktionstasten T1 - T6 erzeugten Steuerbefehle sb1 - sb6 in entsprechende Steuersignale ss1 - ss6 zur Steuerung der Bewegung des Endoskops 3 des Operations-Assistenz-Systems 1 umgesetzt werden, die der Steuereinrichtung 17 zum Antrieb dessen Armsystems 10, 12, 14 um die jeweils zugeordneten Achsen bzw. Gelenke 9, 11, 13, 16 zugeführt werden. Hierbei werden zuvor die durch die einzelnen Arme 10, 12, 14 anzufahrenden Sollpositionen im Bezugskoordinatensystem BKS mit den drei Raumachsen X, Y, Z berechnet.

Das Bedienelement BE weist beispielsweise eine erste Funktionstaste T1 zum Schwenken des auf der Monitoreinheit ME dargestellten Bildes B in Richtung des linken Bildrandes, eine zweite Funktionstaste T2 zum Schwenken des Bildes B in Richtung des rechten Bildrandes sowie eine dritte und vierte Funktionstaste T3, T4 zum Schwenken des Bildes B zum oberen und unteren Bildrand hin auf. Zusätzlich sind eine fünfte und sechste Funktionstaste T5, T6 zum "Zoomen" des dargestellten Bildes B vorgesehen, und zwar wird das Endoskop 3 bei Betätigung der fünften Funktionstaste T5 weiter in den Operationsraum 19 hinein gefahren und bei Betätigung der sechsten Funktionstaste T6 aus diesem heraus geführt. Somit kann der Bediener des Operations-Assistenz-Systems 1 durch entsprechende Betätigung der ersten bis sechsten Funktionstaste T1 - T6 des Bedienelementes BE eine bis zu 360° Drehung des Endoskops 3 innerhalb des Operationsraumes 19 vornehmen, dabei das Endoskop 3 um bis 100° gegenüber der senkrecht durch den Trokarpunkt verlaufende Raumachse z des kartesischen Patientenkoordinatensystems PKS neigen sowie in den Operationsraum 19 bis zu einer Tiefe von ca. 300 mm eindringen.

Um eine hochpräzise Führung des Endoskops 3 mittels der Funktionstasten T1 - T6 zu ermöglichen ist eine genaue Kenntnis der Sollpositionen der einzelnen Bewegungsachsen des Armsystems 10, 12, 14 bezogen auf einen Ausgangspunkt, und zwar den Trokarpunkt T erforderlich. Hierzu werden über einzelne den verschiedenen Bewegungsachsen zugeordnete Sensoren die Koordinaten der Gelenke 9, 11, 13, 15 des Armsystems 10, 12, 14 bezogen auf das Bezugskoordinatensystem BKS im Trokarpunkt T als Ausgangsposition für die weitere Ansteuerung gespeichert. Der Trokarpunkt T bildet zugleich den Ursprung des kartesischen Patientenkoordinatensystems PKS mit den Raumachsen x, y, z, d.h. dem Trokarpunkt T werden die kartesischen Koordinaten XT=0, yr=0, ZT=0 der drei Raumachsen x, y, z des kartesischen Patientenkoordinatensystems PKS zugeordnet.

Die kartesischen Koordinaten x_{T}, y_{T}, z_{T} des ermittelten Trokarpunkts T bilden darüber hinaus zugleich den Mittelpunkt M eines für die Führung des Hilfsinstrumentes bzw. des Endoskops 3 während der Operation, d.h. in einem Operationsbetriebsmodus vorgesehenen Kugelkoordinatensystems aus. Hierbei stimmt der Kugelmittelpunkt M einer durch die jeweiligen Kugelkoordinaten r, Φ, Θ des Kugelkoordinatensystems aufgespannten Kugeloberfläche mit dem Trokarpunkt T überein. Die dem Kamerasystem 2 gegenüberliegende Spitze S des Endoskops 3 liegt hierbei auf der Kugeloberfläche bzw. Teilkugeloberfläche TG, die durch dessen Kugelkoordinaten rs, Φs, Θs aufgespannt wird.

Durch die Kugelkoordinate rs wird der Abstand rs der Spitze S von Trokarpunkt T, d.h. die Eindringtiefe des Endoskops 3 in den Operationsraum 19 angegeben. Die Kugelkoordinate Φs gibt den Drehwinkel um die z-Achse des kartesischen Koordinatensystems an, und zwar in Bezug auf die x-Achse des kartesischen Koordinatensystems im Gegenuhrzeigersinn. Die Kugelkoordinate Θs der Spitze S des Endoskops 3 entspricht hierbei der Summe aus dem Neigungswinkels w der Längsachse L des Endoskops 3 bezogen auf die z-Achse des kartesischen Patientenkoordinatensystems PKS und einem Winkel von +/-180° angegeben.

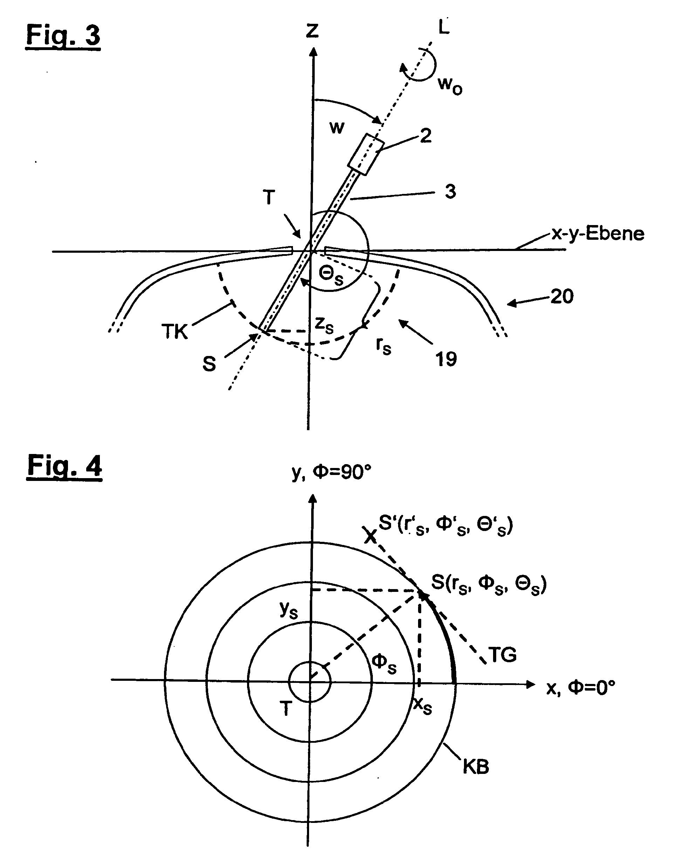

In Figur 3 ist beispielhaft eine schematische Seitenansicht eines in den Operationsraum 19 des Patientenkörpers 20 über den Trokarpunkt T eingeführten Endoskops 3 dargestellt, wobei der Ursprung des kartesischen Patientenkoordinatensystems PKS dem Trokarpunkt T entspricht. In Figur 3 kommt somit die z-Achse des kartesischen Patientenkoordinatensystems PKS in der Zeichenebene zu liegen und die x-y-Ebene des kartesischen Patientenkoordinatensystems PKS verläuft senkrecht zur Zeichenebene. Der Patientenkörper 20 befindet sich auf dem OP-Tisch 5 mit einer zur x-y-Ebene parallelen Liegefläche, wobei die x-y-Ebene durch die Raumachsen x, y des kartesischen Patientenkoordinatensystems PKS aufgespannt wird.

Die Längsachse L des Hilfsinstrumentes bzw. des Endoskops 3 verläuft durch den Trokarpunkt T und somit den Nullpunkt des kartesischen Patientenkoordinatensystems PKS und schließt mit der z- Achse einen Neigungswinkel w ein, und zwar beträgt der Neigungswinkel w im Operationsbetriebsmodus zwischen 0° und 100°. Eine Draufsicht auf die x-y-Ebene des in Figur 3 eingezeichneten kartesischen Patientenkoordinatensystems PKS ist beispielhaft in Figur 4 dargestellt. In Figur 3 und 4 sind sowohl die kartesischen Koordinaten xs, ys, zs der Spitze S des Endoskops 3 im Patientenkoordinatensystem PKS als auch die entsprechenden Kugelkoordinaten rs, Φs, Θs der Spitze S des Endoskops 3 im Kugelkoordinatensystem eingezeichnet.

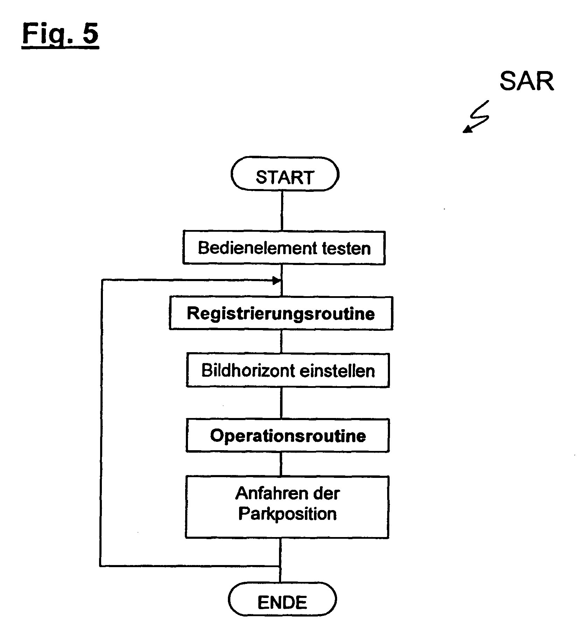

Im Folgenden wird die Steuerung der Führung des chirurgischen Hilfsinstruments 3 mittels des Operations-Assistenz-Systems 1 durch eine in der Steuereinheit CU ausgeführten Steuer- und Auswerteroutine SAR beispielhaft erläutert. In Figur 5 sind beispielhaft die einzelnen Ablaufschritte der Steuer- und Auswertroutine SAR in einem Ablaufdiagramm dargestellt.

Nach dem Start der Steuer- und Auswerteroutine SAR wird die korrekte Funktionsweise des Bedienelementes BE überprüft und im Anschluss daran eine Registrierungsroutine RR zur Registrierung des Operations-Assistenz-Systems 1 ausgeführt. Die Registrierung des Operations-Assistenz-Systems 1 bzw. des Armssystems 10, 12, 14 erfolgt durch Abbildung des kartesischen Bezugskoordinatensystems BKS, welches durch die drei Raumachsen X, Y, Z mit sechs Bewegungsrichtungen +/- X, +/- Y, +/-Z vorgegeben ist, auf den jeweiligen Trokarpunkt T. Hierdurch wird das Patientenkoordinatensystem PKS definiert. Die durch die Betätigung der ersten bis sechsten Funktionstasten T1 - T6 erzeugten Steuerbefehle sb1 - sb6 werden durch die Registrierungsroutine RR in Bewegungen des Armssystems 10, 12, 14 entlang der sechs Bewegungsrichtung +/- X, +/- Y, +/-Z abgebildet.

Nach Durchführung der Registrierungsroutine RR bzw. nach dem Abbilden des Ursprungs des kartesischen Bezugskoordinatensystems BKS in den Trokarpunkt T wird das Armsystem 10, 12, 14 des Operations-Assistenz-Systems 1 beispielsweise in eine Warteposition gefahren.

In der Warteposition oder nach Einführen des Endoskops 3 in den Operationsraum 19 wird der Bildhorizont des auf der Monitoreinheit ME dargestellten Bildes eingestellt, und zwar durch Drehen des Kamerasystems 2 um dessen Längsachse L. Die Einstellung kann entweder manuell oder elektronisch, beispielsweise über eine zugeordnete Antriebseinheit erfolgen. Der eingestellte Drehwinkel bezogen auf die aktuell vorliegende Sollpositionen der Achsen des Armsystems 10, 12, 14 wird in der Speichereinheit MU als Offsetwinkel wo gespeichert. Beispielsweise kann der auf der Monitoreinheit ME darzustellende Rand des Bildes B mittels einer Bildverarbeitungssoftware derart bearbeitet werden, dass im Rahmen der Führung des Endoskops 3 im Operationsraum 19 der Bildhorizont des auf der Monitoreinheit ME dargestellten Bildes B nahezu konstant bleibt.

Nach der Einstellung des Bildhorizontes wird das Operations-Assistenz-Systems 1 in den Operationsbetriebsmodus gesteuert. Hierzu wird durch die Steuer- und Auswerteroutine SAR die Operationsroutine OR ausgeführt. Nach erfolgter Operation wird das Armsystem 10, 12, 14 des Operations-Assistenz-Systems 1 in eine vorgegebene Parkposition gefahren und ggf. die Steuer- und Auswerteroutine SAR beispielsweise in einem unterschiedlichen Trokarpunkt T erneut ausgeführt.

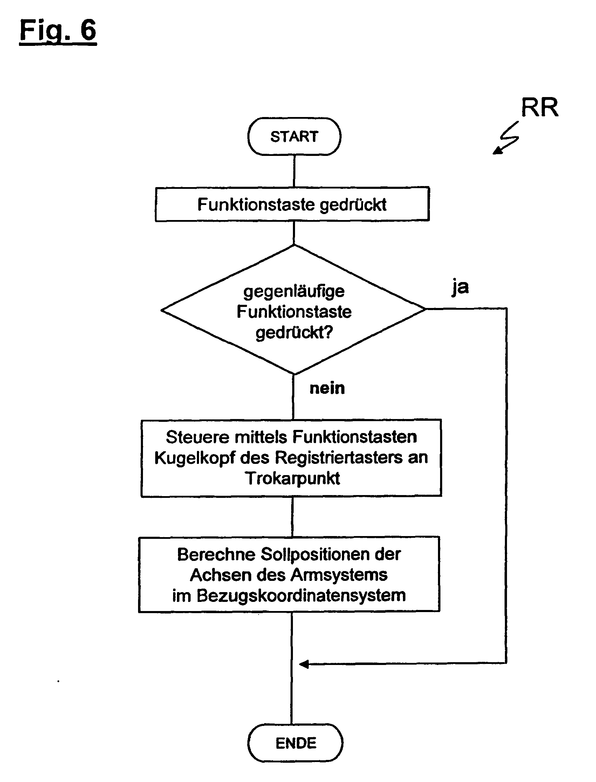

In Figur 6 ist beispielhaft ein Ablaufdiagramm der Registrierungsroutine RR dargestellt. Wird durch Registrierungsroutine RR das Drücken einer der sechs Funktionstasten T1 - T6 erkannt, so wird in einem nächsten Schritt überprüft, ob zugleich eine Betätigung einer gegenläufigen Funktionstaste T1 - T6 vorliegt. Wird beispielsweise die erste und zweite Funktionstaste T1, T2 gleichzeitig betätigt, so erfolgt keine Ansteuerung des Armsystems 10, 12, 14. Hierdurch wird sichergestellt, dass eine defekte Taste oder eine Betätigung gegenläufiger Tasten rechtzeitig durch die Steuer- und Auswerteroutine SAR erkannt wird und durch diese geeignete Sicherheitsmaßnahmen, beispielsweise ein automatisches Abschalten der Antriebe eingeleitet werden können.

Zum Setzen des Trokarpunktes T und somit Registrieren des Operations-Assistenz-Systems 1 wird der Kugelkopf des Registriertasters 18 in den Trokarpunkt T des auf dem OP-Tisch 5 befindlichen Patientenkörpers 20 gesteuert, und zwar durch entsprechende Betätigung der Funktionstasten T1 - T6. Anschließend werden die Sollpositionen der Achsen des Armsystems 10, 12, 14 im kartesischen Bezugskoordinatensystem BKS ausgehend von der kalibrierten Null- oder Ausgangsposition in der Steuer- und Auswerteroutine SAR rechnerisch ermittelt, und zwar unter Verwendung des Offsetvektors V0. Das Operations-Assistenz-System 1 ist nun bezogen auf den Trokarpunkt T registriert.

Im Operationsbetriebsmodus wird in der Steuereinheit CU die Operationsroutine OR ausgeführt. Die Führung des chirurgischen Hilfsinstrumentes, insbesondere des ein Endoskop 3 aufweisendes Kamerasystems 2 erfolgt hierbei vorzugsweise zyklisch, d.h. zu den durch die vorzugsweise digitale Steuereinheit CU vorgegebenen Taktzeiten.

In einem ersten Schritt wird überprüft, ob zumindest eine der Funktionstasten T1 - T6 gedrückt ist. Ist dies der Fall, so wird durch die Operationsroutine OR die Führung der Spitze S des Endoskops 3 ausgehend von der zuletzt gespeicherten Bahnposition entlang der ermittelten Führungsbahn durchgeführt. Wird jedoch durch den Bediener keine der Funktionstasten T1 - T6 gedrückt, so erfolgt eine aktuelle Bestimmung der Koordinaten des Startpunktes für die nachfolgende Bahnberechnung, und zwar der Koordinaten xs, ys, zs der Spitze S des Hilfsinstruments bzw. Endoskops 3 im kartesischen Patientenkoordinatensystem PKS. Die aktuell ermittelten oder bereits gespeicherten Koordinaten xs, ys, zs der Spitze S des Endoskops 3 werden anschließend in die zugehörigen Kugelkoordinaten rs, Φs, Θs des zugeordneten Kugelkoordinatensystem umgerechnet. Die erforderlichen Umrechnungsformeln sind hinlänglich aus dem Stand der Technik bekannt.

Abhängig von den ermittelten Kugelkoordinaten rs, Φs, Θs der Spitze S wird der Neigungswinkel w des Endoskops 3 bezogen auf die *z*-Achse des kartesischen Patientenkoordinatensystems PKS bestimmt, welcher ca. zwischen 0° und 100° aufweisen kann. Der ermittelte Neigungswinkel w wird mit einem ausgewählten, in der Speichereinheit MU hinterlegten Neigungssollwinkel ws verglichen, der im Bereich zwischen 10° und 25°, vorzugsweise zwischen 15° und 20° liegt.

Überschreitet der Neigungswinkel w den Neigungssollwinkel ws, so wird die Spitze S des Endoskops 3 auf einer durch die Kugelkoordinaten rs, Φs, Θs aufgespannten Teilkugeloberfläche TK verlaufenden Bahn, vorzugsweise einer Kreisbahn KB geführt. Abhängig von der Betätigung der ersten bis vierten Funktionstaste T1 bis T4 erfolgt eine entsprechende Nachführung der Spitze S in die jeweils ausgewählte Bewegungsrichtung auf der Teilkugeloberfläche TK bzw. der jeweiligen Kreisbahn KB. Im Rahmen der Kugelsteuerung bewegt sich die Spitze S des Endoskops 3 auf Kreisbahnen ähnlichen Längen- und Breitengrads auf der im Operationsraum 19 aufgespannten Teilkugeloberfläche TK, deren Ebenen senkrecht bzw. parallel zur x-y-Ebene angeordnet sind. Eine Betätigung des ersten oder zweiten Funktionstasters T1, T2 entspricht einer Bewegung nach links oder rechts auf einer konzentrisch um die z-Achse verlaufenden Kreisbahn KB (siehe Figur 4) und somit annähernd einer Links- oder Rechtsbewegung des auf der Monitoreinheit ME dargestellten Bildes B. Analog hierzu entspricht eine Betätigung des dritten oder vierten Funktionstasters T3, T4 einer Bewegung nach oben oder nach unten auf einer senkrecht zur x-y-Ebene verlaufenden Kreisbahn KB.

Unterschreitet der ermittelte Neigungswinkel w den Neigungssollwinkel ws, so wird die Spitze S des Endoskops 3 entlang einer durch die Spitze S des Endoskops 3 verlaufenden und an der Teilkugeloberfläche TK anliegenden Tangente TG geführt. Im Rahmen der Tangentensteuerung wird die Spitze S des Endoskops 3 abhängig von der aktuellen Position der Spitze S, die auch die Bilddrehung und somit den Bildhorizont bestimmt, tangential zu der im Operationsraum 19 aufgespannten Teilkugeloberfläche TK, und zwar entlang der Richtung der beiden Hauptachsen des gedrehten Bildes B bewegt. Die Bewegungen der Spitze S beschreiben bei einer kontinuierlichen Ansteuerung nach links oder rechts, d.h. einer kontinuierlichen Betätigung der ersten oder zweiten Funktionstaste T1, T2 keine Kreisbahn, sondern eine spiralförmige Bewegungsbahn. Die Steuerung nach oben oder unten erfolgt analog zur Kugelsteuerung auf einer senkrecht zur x-y-Ebene verlaufenden Kreisbahn.

Somit ergeben sich zumindest zwei unterschiedliche Ansteuerungsarten zur Ermittlung einer neuen Sollposition, d.h. der mittels des Antriebssystems anzufahrenden aktualisierten Kugelkoordinaten r's, Φ's, Θ's der Spitze S des Endoskops 3 auf einer durch die Ansteuerungsart vorgegebenen Bewegungsbahn, d.h. abhängig von ermittelten Neigungswinkel w wird die aufgrund der Betätigung einer der ersten bis vierten Funktionstaste T1 bis T4 ermittelte Bewegungsbahn unterschiedlich berechnet. Die neue Sollposition bzw. die anzufahrenden aktualisierten Kugelkoordinaten r's, Φ's, Θ's werden ggf. in der Speichereinheit MU hinterlegt.

Eine Betätigung der fünften und sechsten Funktionstaste T5, T6 bewirkt in beiden Fällen beispielsweise eine Verkleinerung bzw. Vergrößerung des Radius rs der Teilkugel. Die Ermittlung der aktualisierten Kugelkoordinaten r's, Φ's, Θ's der Spitze S auf der vorgegeben Bewegungsbahn ist darüber hinaus abhängig von der Art und Dauer der Betätigung der ersten bis vierten Funktionstaste T1 bis T4.

Anschließend werden zu den aktualisierten Kugelkoordinaten r's, Φ's, Θ's der Spitze S' die aktualisierten Koordinaten x's, y's, z's der Spitze S' im kartesischen Patientenkoordinatensystem PKS ermittelt und in aktualisierte Koordinaten X's, Y's, Z's der Spitze S' im kartesischen Bezugskoordinatensystem BKS umgerechnet. Ausgehend hiervon werden die jeweiligen Achsgeschwindigkeitssollwerte V zur Ansteuerung der Antriebe des Armsystems 10, 12, 14 bestimmt. Ggf. wird abhängig vom gespeicherten Offsetwinkel wo zum Ausgleich des Bildhorizonts der Drehwinkel des Kamerasystems 2 um die Längsachse L nachgezogen.

Die Erfindung wurde voranstehend an einem Ausführungsbeispiel beschrieben. Es versteht sich, dass zahlreiche Änderungen sowie Abwandlungen möglich sind, ohne das dadurch der der Erfindung zugrunde liegende Erfindungsgedanke verlassen wird.

Beispielsweise besteht die die Möglichkeit mehrere Trokarpunkte T zu setzen und zwischen diesen zu wechseln. Ferner kann zur Gewährleistung der Sicherheit des Operations-Assistenz-Systems 1 zusätzlich zum Ausschluss einer gegenläufigen Betätigung der Funktionstasten T1 - T6 mittels eines zentralen Energieschalters die zum Betrieb der Antriebe erforderliche Energiezufuhr zentral geschaltet werden.

Auch kann die Steuer- und Auswerteroutine SAR eine Bildverarbeitungsroutine zur optischen Erkennung einer drohenden Kollision der Spitze S des Endoskops 3 mit dem im Operationsraum 19 vorhandenen Gewebe vorgesehen sein, welche beispielsweise die im erzeugten Bild vorhandenen Hell/Dunkelkontraste und deren zeitliche Änderung auswertet.

### Bezugszeichenliste

- 1: Operations-Assistenz-System
- 2: Kamerasystem
- 3: Endoskop
- 4: Gehäuse
- 5: OP-Tisch
- 6: Klemmvorrichtung
- 7: Schwenkhebel
- 8: Tragsäule
- 9: Gelenk
- 10: Arm
- 10.1: Armoberseite
- 10.2: Armunterseite
- 11: Gelenk
- 12: Arm
- 12.1: Armoberseite
- 12.2: Armunterseite
- 13: Gelenk
- 14: Werkzeugträger
- 14.1: kürzerer Schenkel
- 14.2: längerer Schenkel
- 15: Halter
- 16: Gelenk
- 17: Steuereinrichtung
- 18: Registriertaster
- 19: Operationsraum
- 20: Patientenkörper

- B: Bild
- BE: Bedienelement
- BKS: kartesisches Bezugskoordinatensystem
- CS: Computersystem
- CU: Steuereinheit
- L: Längsachse
- ME: Monitoreinheit
- MU: Speichereinheit
- OR: Operationsroutine
- PKS: kartesisches Patientenkoordinatensystem
- r's, Φ's, Θ's: aktualisierte Kugelkoordinaten der verschobenen Spitze
- RR: Registrierungsroutine
- rs, Φs, Θs: Kugelkoordinaten der Spitze
- S, S': Spitze
- SAR: Steuer- und Auswerteroutine
- sb1 - sb6: Steuerbefehle
- ss1 - ss6: Steuersignale
- T: Trokarpunkt
- T1 - T6: erster bis sechste Funktionstaste
- TG: Tangente
- TK: Teilkugeloberfläche
- V: Achsgeschwindigkeitssollwerte
- V0: Offsetvektor
- VA: vertikale Achse
- w: Neigungswinkel
- wo: Offsetwinkel
- ws: Sollneigungswinkel
- X, Y, Z: Koordinaten des Achsen im kartesischen Bezugskoordinatensystem
- x, y, z: Koordinaten des kartesischen Patientenkoordinatensystems
- X', Y', Z': aktualisierte Koordinaten des kartesischen Bezugskoordinatensystem
- x's, y's, z's: aktualisierte kartesische Koordinaten der verschobenen Spitze
- X0, Y0 Z0: Koordinaten des Offsetvektors
- xs, ys, zs: kartesische Koordinaten der Spitze
- x_{T}, y_{T}, z_{T}: kartesische Koordinaten des Trokarpunktes

## Patentansprüche

1. Operations-Assistenz-System zur Führung eines chirurgischen Hilfsinstrumentes, insbesondere eines ein Endoskop (3) aufweisendes Kamerasystems (2), mit einem eine Instrumentenhalterung (15) aufweisenden Armsystem (10, 12, 14) und einer zu dessen Ansteuerung vorgesehenen Steuereinrichtung (17), die an ein mit einem Bedienelement (BE) verbundenen Computersystem (CS) angeschlossen ist, das zur von der manuellen Betätigung zumindest einer Funktionstaste (T1 - T6) des Bedienelements (BE) abhängigen Führung des chirurgischen Hilfsinstrumentes (3) ausgebildet ist, wobei zumindest die Spitze (3) des chirurgischen Hilfsinstruments (3) mittels des Armsystems (10, 12, 14) in einem kartesischen Patientenkoordinatensystem (PKS) gesteuert bewegbar ist und zumindest eine der drei Raumachsen (x, y, z) des kartesischen Patientenkoordinatensystem (PKS) durch die das chirurgische Hilfsinstrument (3) aufnehmende Operationsöffnung oder den Trokarpunkt (T) verläuft,
**dadurch gekennzeichnet,**
**dass** eine Steuer- und Auswerteroutine (SAR) vorgesehen ist, die zur Ermittlung der Neigungswinkel (w) des chirurgischen Hilfsinstrumentes (3) in Bezug auf die durch den Trokarpunkt (T) verlaufende Raumachse (z) des kartesischen Patientenkoordinatensystem (PKS) und zum Vergleich der ermittelte Neigungswinkel (w) mit einem vorgegebenen Sollneigungswinkel (ws) ausgebildet ist, wobei bei einem Überschreiten des Sollneigungswinkels (ws) durch den ermittelten Neigungswinkel (w) die Spitze (S) des chirurgischen Hilfsinstrumentes (3) auf einer die Spitze (S) aufnehmenden Teilkugeloberfläche (TK) geführt wird und bei einem Unterschreiten des Sollneigungswinkel (ws) durch den ermittelten Neigungswinkel (w) die Spitze (S) des chirurgischen Hilfsinstrumentes (3) entlang einer durch die Spitze (S) des Hilfsinstrumentes (3) verlaufenden Tangente (TG) an die Teilkugeloberfläche (TK) geführt wird.

2. Operations-Assistenz-System nach Anspruch 1, **dadurch gekennzeichnet, dass** bei einem Überschreiten des Sollneigungswinkels (ws) durch den ermittelten Neigungswinkel (w) die Spitze (S) des Hilfsinstrumentes (3) auf einer den Trokarpunkt (T) konzentrisch umgebenden Kreisbahn (KB) geführt wird und/oder dass dem Trokarpunkt (T) die Koordinaten (x_{T}, y_{T}, z_{T}) des Nullpunktes oder des Ursprungs des kartesischen Patientenkoordinatensystem (PKS) zugeordnet werden.

3. Operations-Assistenz-System nach Anspruch 1 oder 2, **dadurch gekennzeichnet, dass** zur Registrierung des Operations-Assistenz-Systems (1) ein am Armsystem (10, 12, 14) starr angeordneter Registriertaster (18) an den Trokarpunkt (T) gesteuert wird und die Koordinaten (X, Y, Z) der Achsen des Armsystems (10, 12, 14) in einem kartesischen Bezugskoordinatensystem (BKS) bestimmt werden.

4. Operations-Assistenz-System nach Anspruch 3, **dadurch gekennzeichnet, dass** zur Berechnung des Startpunktes der Führung die Koordinaten (X, Y, Z) der Achsen des Armsystems (10, 12, 14) in dem kartesischen Bezugskoordinatensystem (BKS) bestimmt werden und abhängig davon die aktuellen kartesischen Koordinaten (xs, ys, zs) der Spitze (S) des Hilfsinstrumentes (3) bestimmt werden.

5. Operations-Assistenz-System nach Anspruch 4, **dadurch gekennzeichnet, dass** abhängig von den kartesischen Koordinaten (xs, ys, zs) der Spitze (S) des Hilfsinstrumentes (3) im kartesischen Patientenkoordinatensystem (PKS) die zugehörigen Kugelkoordinaten (rs, Φs, Θs) der Spitze (S) des Hilfsinstrumentes (3) bestimmt werden.

6. Operations-Assistenz-System nach Anspruch 5, **dadurch gekennzeichnet, dass** der Neigungswinkel (w) durch Auswertung zumindest einer der Kugelkoordinaten (rs, Φs, Θs) der Spitze (S) des Hilfsinstrumentes (3) bestimmt wird und/oder dass die mittels des Armsystems (10, 12, 14) des Operations-Assistenz-Systems (1) anzufahrende Sollposition der Spitze (S') des Hilfsinstrumentes (3), die abhängig vom ermittelten Neigungswinkel (w) auf der Teilkugeloberfläche (TK) oder Tangente (TG) liegt, bestimmt wird.

7. Operations-Assistenz-System nach Anspruch 6, **dadurch gekennzeichnet, dass** die anzufahrende Sollposition der Spitze (S') des Hilfsinstrumentes (3) in Form von aktualisierten Kugelkoordinaten (r's, Φ's, Θ's) bestimmt wird.

8. Operations-Assistenz-System nach Anspruch 7, **dadurch gekennzeichnet, dass** die aktualisierten Kugelkoordinaten (r's, Φ's, Θ's) der Spitze (S') des Hilfsinstrumentes (3) zunächst in aktualisierte kartesischen Koordinaten (x's, y's, z's) des Patientenkoordinatensystems (PKS) umgerechnet werden, aus den die zur Ansteuerung des Armsystem (10, 12, 14) des Operations-Assistenz-Systems (1) erforderlichen aktualisierten kartesischen Koordinaten (X', Y', Z') der Achsen des Armsystems (10, 12, 14) im Bezugskoordinatensystems (BKS) abgeleitet werden.

9. Operations-Assistenz-System nach einem der vorhergehenden Ansprüche,
**dadurch gekennzeichnet, dass** das Operations-Assistenz-Systems (1) mittels einer Registrierungsroutine (RR) in zumindest einem Trokarpunkt (T) registriert wird.

10. Operations-Assistenz-System nach Anspruch 9, **dadurch gekennzeichnet, dass** abhängig von den aktualisierten kartesischen Koordinaten (X', Y', Z') der Achsen des Armsystems (10, 12, 14) zugehörige Achsgeschwindigkeitssollwerte (V) bestimmt werden.

11. Operations-Assistenz-System nach einem der vorhergehenden Ansprüche,
**dadurch gekennzeichnet, dass** das Hilfsinstrument (3) derart über die Operationsöffnung in den Patientenkörper (20) eingeführt wird, dass die Längsachse (L) des Hilfsinstrumentes (3) durch den Nullpunkt des kartesischen Patientenkoordinatensystems (PKS) verläuft und mit der *z*-Achse den Neigungswinkel (w) einschließt.

12. Operations-Assistenz-System nach einem der vorhergehenden Ansprüche,
**dadurch gekennzeichnet, dass** mittels dem Kamerasystem (2) Bilder (B) erzeugt werden, welche auf einer Monitoreinheit (ME) dargestellt werden.

13. Operations-Assistenz-System nach Anspruch 11, **dadurch gekennzeichnet, dass** der Bildhorizont der auf der Monitoreinheit (ME) dargestellten Bilder (B) vor einer minimal invasiven Operation eingestellt wird, und zwar durch Drehen des Kamerasystems (2) um dessen Längsachse (L).

14. Operations-Assistenz-System nach Anspruch 12, **dadurch gekennzeichnet, dass** der Bildhorizont der auf der Monitoreinheit ME dargestellten Bilder (B) während der minimal invasiven Operation nahezu konstant gehalten wird.

15. Operations-Assistenz-System nach einem Ansprüche 2 bis 12, **dadurch gekennzeichnet, dass** durch eine Betätigung der ersten oder zweiten Funktionstaste (T1, T2) die Spitze (S) auf einer konzentrisch um die *z*-Achse (z) verlaufenden Kreisbahn (KB) nach links oder rechts geführt wird und hierdurch eine Links- oder Rechtsbewegung des auf der Monitoreinheit (ME) dargestellten Bildes (B) bewirkt wird und/oder dass der Sollneigungswinkels (ws) zwischen 10° und 25°, vorzugsweise zwischen 15° und 20° gewählt wird.

## Claims

1. Surgery assistance system for guiding a surgical instrument, more particularly a camera system (2) provided with an endoscope (3), comprising an arm system (10, 12, 14) containing an instrument holder (15), and a control mechanism (17) provided for the control thereof, which is linked to a computer system (CS) which is connected to an operating element (BE) and is designed for guiding the surgical instrument (3) dependent on the manual actuation of at least one function key (T1-T6) of the operating element (BE), wherein at least the tip (3) of the surgical instrument (3) can be moved controlled by means of the arm system (10,1 2, 14) in a Cartesian patient coordinate system (PKS) and at least one of the three spatial axes (x, y, z) of the Cartesian patent coordinate system (PKS) runs through the trocar point (T) or operating aperture receiving the surgical instrument (3),
**characterised in that**
a control and evaluating routine (SAR) is provided which is designed to determine the angle of inclination (w) of the surgical instrument (3) in relation to the spatial axis (z) of the Cartesian patient coordinate system (PKS) running through the trocar point (T) and to compare the determined angle of inclination (w) with a predetermined ideal angle of inclination (ws) wherein when the determined angle of inclination (w) is greater than the ideal angle of inclination (ws) the tip (S) of the surgical instrument (3) is guided on a semi-spherical surface (TK) which receives the tip (S) and when the determined angle of inclination (w) is below the ideal angle of inclination (ws) the tip (S) of the surgical instrument (3) is guided along a tangent (TG) which extends through the tip (S) of the instrument (3) and rests against the semi-spherical surface (TK).

2. Surgery assistance system according to claim 1 **characterised in that** when the determined angle of inclination (w) is greater than the ideal angle of inclination (ws) the tip (S) of the instrument (3) is guided on a circular path (KB) which surrounds the trocar point (T) concentrically and/or that the coordinates (xr, yr, zr) of the zero point or point of origin of the Cartesian patient coordinate system (PKS) are assigned to the trocar point (T).

3. Surgery assistance system according to claim 1 or 2 **characterised in that** for registering the surgery assistance system (1) a registering scanner (18) mounted rigid on the arm system (10, 12, 14) is controlled at the trocar point (T) and the coordinates (X, Y, Z) of the axes of the arm system (10, 12, 14) are determined in a Cartesian reference coordinate system (BKS).

4. Surgery assistance system according to claim 3 **characterised in that** to calculate the start point of the guidance the coordinates (X, Y, Z) of the axes of the arm system (10, 12, 14) are determined in the Cartesian reference coordinate system (BKS) and the actual Cartesian coordinates (xs, ys, zs) of the tip (S) of the instrument (3) are determined in dependence thereon.

5. Surgery assistance system according to claim 4 **characterised in that** the associated spherical coordinates (rs, Φs, ∂s) of the tip (S) of the instrument (3) are determined in dependence on the Cartesian coordinates (xs, ys, zs) of the tip (S) of the instrument (3) in the Cartesian patient coordinate system (PKS).

6. Surgery assistance system according to claim 5 **characterised in that** the angle of inclination (w) is determined by evaluating at least one of the spherical coordinates (rs, Φs, ∂s) of the tip (S) of the instrument (3) and/or that the ideal position is determined of the tip (S') of the instrument (3) which is to be arrived at by means of the arm system (10, 12, 14) of the surgery assistance system (1) which lies on the semi spherical surface (TK) or tangent (TG) dependent on the determined angle of inclination (w).

7. Surgery assistance system according to claim 6 **characterised in that** the ideal position of the tip (S') of the instrument (3) which is to be reached is determined in the form of updated spherical coordinates (r's, Φ's, ∂'s).

8. Surgery assistance system according to claim 7 **characterised in that** the updated spherical coordinates (r's, Φ's, ∂'s) of the tip (S') of the instrument (3) are initially converted into updated Cartesian coordinates (x's, y's, z's) of the patient coordinate system (PKS) from which are derived the updated Cartesian coordinates (X', Y', Z') of the axes of the arm system (10, 12, 14) required for controlling the arm system (10, 12, 14) of the surgery assistance system (1) in the reference coordinate system (BKS).

9. Surgery assistance system according to one of the preceding claims **characterised in that** the surgery assistance system (1) is registered by means of a registration routine (RR) in at least one trocar point.

10. Surgery assistance system according to claim 9 **characterised in that** associated axial speed ideal values (V) are determined in dependence on the updated Cartesian coordinates (X', Y', Z') of the axes of the arm system (10, 12, 14).

11. Surgery assistance system according to one of the preceding claims **characterised in that** the instrument (3) is introduced into the body (20) of the patient via the operation aperture in such a way that the longitudinal axis (L) of the instrument (3) extends through the zero point of the Cartesian patient coordinate system (PKS) and includes the angle of inclination (w) with the z-axis.

12. Surgery assistance system according to one of the preceding claims **characterised in that** images (B) are produced by means of the camera system (2) and are displayed on a monitor unit (ME).

13. Surgery assistance system according to claim 11 **characterised in that** the image horizon of the images (B) displayed on the monitor unit (ME) is set prior to a minimal invasive operation, namely by rotating the camera system (2) about its longitudinal axis (L).

14. Surgery assistance system according to claim 12 **characterised in that** the image horizon of the images (B) displayed on the monitor unit (ME) is kept practically constant during the minimal invasive operation.

15. Surgery assistance system according to one of claims 2 to 12 **characterised in that** by actuating the first or second function key (T1, T2) the tip (S) is guided to the left or right on a circular path (KB) which runs concentrically round the z-axis (z) and hereby actuates a left or right movement of the image (B) displayed on the monitor unit (ME) and/or that the ideal angle of inclination (ws) is selected between 10° and 25°, preferably between 15° and 20°.

## Revendications

1. Système d'assistance d'opération pour le guidage d'un instrument d'aide chirurgicale, en particulier d'un système de caméra (2) présentant un endoscope (3), doté d'un système de bras (10, 12, 14) présentant un support d'instrument (15) et d'un dispositif de commande (17) prévu pour son actionnement, qui est raccordé à un système informatique (CS) relié à un élément de commande (BE), lequel est conçu pour le guidage, dépendant de la commande manuelle d'au moins une touche de fonction (T1-T6) de l'élément de commande (BE), de l'instrument d'aide chirurgicale (3), au moins la pointe (3) de l'instrument d'aide chirurgical (3) pouvant être déplacée de façon commandée au moyen du système de bras (10, 12, 14) dans un système cartésien de coordonnées de patient (PKS) et au moins l'un des trois axes d'espace (x, y, z) du système cartésien de coordonnées de patient (PKS) passant par l'ouverture d'opération recevant l'instrument d'aide chirurgicale (3) ou le point de trocart (T), **caractérisé en ce que** :
il est prévu une routine de commande et d'analyse (SAR), qui est conçue pour la détermination des angles d'inclinaison (w) de l'instrument d'aide chirurgicale (3) par rapport à l'axe d'espace (z), passant par le point de trocart (T), du système cartésien de coordonnées de patient (PKS) et pour la comparaison des angles d'inclinaison (w) déterminés avec un angle d'inclinaison théorique prédéfini (ws), la pointe (S) de l'instrument d'aide chirurgicale (3) étant guidée sur une surface de sphère partielle (TK) recevant la pointe (S) dans le cas d'un dépassement de l'angle d'inclinaison théorique (ws) par l'angle d'inclinaison déterminé (w) et la pointe (S) de l'instrument d'aide chirurgicale (3) étant guidée le long d'une tangente (TG), passant par la pointe (S) de l'instrument d'aide (3), à la surface de sphère partielle (TK) dans le cas d'un dépassement de l'angle d'inclinaison théorique (ws) par l'angle d'inclinaison déterminé (w).

2. Système d'assistance d'opération selon la revendication 1, **caractérisé en ce que**, dans le cas d'un dépassement de l'angle d'inclinaison théorique (ws) par l'angle d'inclinaison déterminé (w), la pointe (S) de l'instrument d'aide (3) est guidée sur une trajectoire circulaire (KB) entourant le point de trocart (T) de façon concentrique et/ou **en ce que** les coordonnées (xT, yT, zT) du point zéro ou de l'origine du système cartésien de coordonnées de patient (PKS) sont attribuées au point de trocart (T).

3. Système d'assistance d'opération selon la revendication 1 ou 2, **caractérisé en ce qu'**un bouton-poussoir d'enregistrement (18) disposé de façon rigide sur le système de bras (10, 12, 14) est commandé sur le point de trocart (T) pour l'enregistrement du système d'assistance d'opération (1) et les coordonnées (X, Y, Z) des axes du système de bras (10, 12, 14) sont déterminées dans un système cartésien de coordonnées de référence (BKS).

4. Système d'assistance d'opération selon la revendication 3, **caractérisé en ce que** les coordonnées (X, Y, Z) des axes du système de bras (10, 12, 14) sont déterminées dans le système cartésien de coordonnées de référence (BKS) pour le calcul du point de démarrage et les coordonnées cartésiennes actuelles (xs, ys, zs) de la pointe (S) de l'instrument d'aide (3) sont déterminées en fonction de cela.

5. Système d'assistance d'opération selon la revendication 4, **caractérisé en ce que** les coordonnées de sphère spécifiques (rs, Φs, Θs) de la pointe (S) de l'instrument d'aide (3) sont déterminées en fonction des coordonnées cartésiennes (xs, ys, zs) de la pointe (S) de l'instrument d'aide (3) dans le système cartésien de coordonnées de patient (PKS).

6. Système d'assistance d'opération selon la revendication 5, **caractérisé en ce que** l'angle d'inclinaison (w) est déterminé par l'analyse d'au moins l'une des coordonnées de sphère (rs, Φs, Θs) de la pointe (S) de l'instrument d'aide (3) et/ou **en ce que** la position théorique, à atteindre au moyen du système de bras (10, 12, 14) du système d'assistance d'opération (1), de la pointe (S') de l'instrument d'aide (3), qui se situe en fonction de l'angle d'inclinaison déterminé (w) sur la surface de sphère partielle (TK) ou sur la tangente (TG), est déterminée.

7. Système d'assistance d'opération selon la revendication 6, **caractérisé en ce que** la position théorique à atteindre de la pointe (S') de l'instrument d'aide (3) est déterminée sous la forme de coordonnées de sphère actualisées (r's, Φ's, Θ's).

8. Système d'assistance d'opération selon la revendication 7, **caractérisé en ce que** les coordonnées de sphère actualisées (r's, Φ's, Θ's) de la pointe (S') de l'instrument d'aide (3) sont converties d'abord en coordonnées cartésiennes actualisées (x's, y's, z's) du système de coordonnées de patient (PKS), à partir desquelles les coordonnées cartésiennes actualisées (X', Y', Z'), nécessaires pour l'actionnement du système de bras (10, 12, 14) du système d'assistance d'opération (1), des axes du système de bras (10, 12, 14) sont déduites dans le système de coordonnées de référence (BKS).

9. Système d'assistance d'opération selon l'une quelconque des revendications précédentes, **caractérisé en ce que** le système d'assistance d'opération (1) est enregistré au moyen d'une routine d'enregistrement (RR) dans au moins un point de trocart (T).

10. Système d'assistance d'opération selon la revendication 9, **caractérisé en ce que** des valeurs théoriques de vitesse d'axe (V) spécifiques sont déterminées en fonction des coordonnées cartésiennes (X', Y', Z') actualisées des axes du système de bras (10, 12, 14).

11. Système d'assistance d'opération selon l'une quelconque des revendications précédentes, **caractérisé en ce que** l'instrument d'aide (3) est introduit par l'ouverture d'opération dans le corps de patient (20), de telle sorte que l'axe longitudinal (L) de l'instrument d'aide (3) passe par le point zéro du système cartésien de coordonnées de patient (PKS) et forme l'angle d'inclinaison (w) avec l'axe z.

12. Système d'assistance d'opération selon l'une quelconque des revendications précédentes, **caractérisé en ce que** des images (B), qui sont représentées sur une unité formant moniteur (ME), sont engendrées au moyen du système de caméra (2).

13. Système d'assistance d'opération selon la revendication 11, **caractérisé en ce que** l'horizon de l'image des images (B) représentées sur l'unité formant moniteur (ME) est réglé avant une opération invasive de façon minimale, et ce par rotation du système de caméra (2) autour de son axe longitudinal (L).

14. Système d'assistance d'opération selon la revendication 12, **caractérisé en ce que** l'horizon de l'image des images (B) représentées sur l'unité formant moniteur (ME) est maintenu pratiquement constant pendant l'opération invasive de façon minimale.

15. Système d'assistance d'opération selon l'une quelconque des revendications 2 à 12, **caractérisé en ce que** la pointe (S) est guidée sur une trajectoire circulaire (KB) passant de façon concentrique autour de l'axe z (z) vers la gauche ou vers la droite par un actionnement de la première ou de la seconde touche de fonction (T1, T2), et un mouvement à gauche ou à droite de l'image (B) représentée sur l'unité formant moniteur (ME) est effectué et/ou **en ce que** l'angle d'inclinaison théorique (ws) est choisi entre 10° et 25°, de préférence entre 15° et 20°.
